# EUROPÄISCHE PATENTSCHRIFT

(11) **EP 1 805 266 B1**
(45) Veröffentlichungstag und Bekanntmachung des Hinweises auf die Patenterteilung: **26.08.2009**
(21) Anmeldenummer: 05771747.2
(22) Anmeldetag: 11.07.2005
(51) Int. Cl.: C09B 56/18

(54) **KATIONISCHE AZOAZINFARBSTOFFE UND DIESE VERBINDUNGEN ENTHALTENDE FÄRBEMITTEL**
CATIONIC AZOAZINIC DYES AND COOLANTS CONTAINING SAID COMPOUNDS
COLORANTS AZOAZINIQUES CATIONIQUES, ET AGENTS DE COLORATION CONTENANT CES COMPOSES

(30) Priorität: 29.10.2004 DE 102004052608
(43) Veröffentlichungstag der Anmeldung: 11.07.2007
(73) Patentinhaber: The Procter and Gamble Company, Cincinnati, Ohio 45202 (US)
(72) Erfinder: SPECKBACHER, Markus, 63739 Aschaffenburg (DE); BRAUN, Hans Jürgen, CH-3182 Ueberstorf (CH)
(74) Vertreter: Kohol, Sonia
(86) Internationale Anmeldenummer: PCT/EP2005/007484
(87) Internationale Veröffentlichungsnummer: WO 2006/048057

(56) Entgegenhaltungen:
- WO-A-03/060015
- WO-A-20/04083312
- GB-A- 974 345

## Beschreibung

Gegenstand der vorliegenden Erfindung sind neuartige kationische Azoazinfarbstoffe, sowie diese Verbindungen enthaltene Färbemittel für Fasern, insbesondere Keratinfasern, wie zum Beispiel menschliche Haare.

Aus den Dokumenten WO03/060015 A und WO/2004/083312 A sind kationische Hydrazonfarbstoffe und/oder Azofarbstoffe bekannt, die zum Färben von organischen Materialien, insbesondere menschlichen Haaren, eingesetzt werden können.

Für das Färben von Fasermaterialien, insbesondere von keratinhaltigen Fasern, beispielsweise Haaren, Wolle oder Pelzen, kommen im allgemeinen entweder Oxidationsfarbstoffe, die durch oxidative Kupplung einer oder mehrerer Entwicklerkomponenten mit einer oder mehreren Kupplerkomponenten entstehen, oder direktziehende Farbstoffe zur Anwendung. Bei Bedarf können dem oxidativen System oxidationsstabile, direktziehende Farbstoffe zugesetzt werden, um besondere Farbeffekte zu erzielen. Direktziehende Farbstoffe werden in geeignete Trägermassen eingearbeitet, um dann auf die Faser aufgebracht zu werden. Dieses Verfahren, allgemein als Tönung bekannt, ist einfach anzuwenden, ausgesprochen mild und zeichnet sich durch eine geringe Schädigung der Keratinfaser aus, wenn kein Ammoniak oder Peroxid zugesetzt wird. Die hierbei verwendeten Farbstoffe müssen allerdings einige Anforderungen erfüllen. So müssen sie in toxikologischer und dermatologischer Hinsicht unbedenklich sein und die Erzielung von Färbungen in der gewünschten Intensität und Brillianz ermöglichen. Ausserdem wird für die erzielten Färbungen eine gute Lichtechtheit, Resistenz gegen Shampoonieren und Conditioners, sowie eine gute Reibechtheit gefordert.

Für ein direktziehendes, nicht-oxidatives Färbemittel für Keratinfasern wird in der Regel eine Kombination von verschiedenen nicht-oxidativen

Farbstoffen benötigt, um bestimmte Nuancierungen zu erreichen. Da die Auswahl an derartigen Farbstoffen, die die genannten Anforderungen hinreichend erfüllen, beschränkt ist, besteht weiterhin großer Bedarf an solchen Farbstoffen.

Aufgabe der vorliegenden Erfindung ist es, direktziehende Farbstoffe zum Färben von Keratinfasern, insbesondere von menschlichen Haaren, bereitzustellen, die diesen Anforderungen genügen.

Überraschenderweise wurde nunmehr gefunden, dass kationische Azoazinfarbstoffe der allgemeinen Formel (I) als direktziehende Farbstoffe in Färbemassen ohne zugesetztes Oxidationsmittel sehr schonend auf Keratinfasern aufgebracht werden können. Da diese Farbstoffe gegenüber Oxidationsmitteln stabil sind, können sie aber auch in Oxidationsmittel, beispielsweise Peroxide, enthaltenden aufhellenden Färbemitteln verwendet werden.

Gegenstand der vorliegenden Erfindung sind daher kationische Farbstoffe der allgemeinen Formel (I),

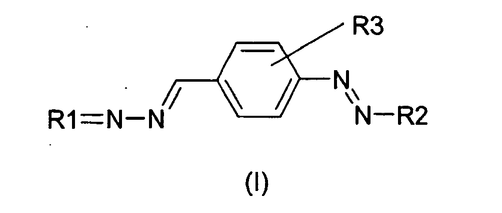

**R1** ausgewählt ist aus Resten der Formeln (II), (III), (IV), (V) und (VI),

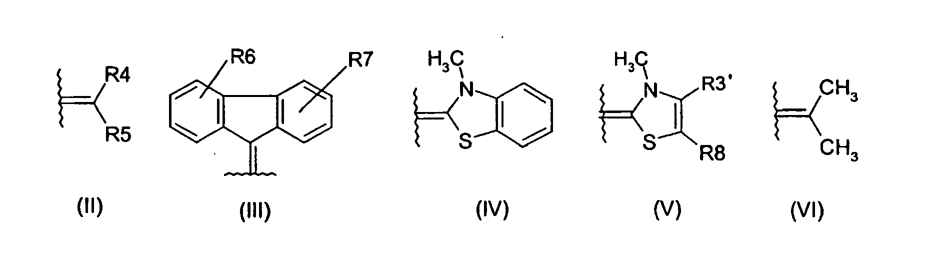

**R2, R4** und **R5** gleich oder verschieden sein können und unabhängig voneinander ausgewählt sind aus Resten der Formeln (VII), (VIII) und (IX);

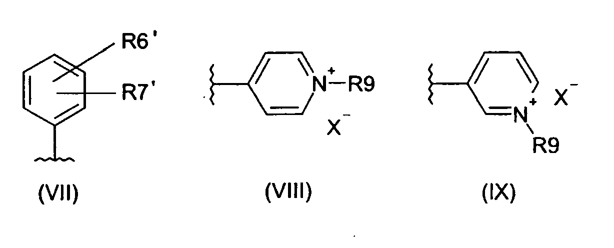

**R3, R3',R6, R6', R7, R7'** und **R8** gleich oder verschieden sein können und unabhängig voneinander gleich Wasserstoff, einer C₁-C₆-Alkylaminogruppe, einer C₁-C₆-*N,N*-Dialkylaminogruppe, einer C₁-C₆-*N,N*-Dihydroxyalkylaminogruppe, einer C₁-C₆-*N*-hydroxyalkyl-*N*-alkylaminogruppe, einer C₁-C₆-Alkylcyanogruppe, einer Nitrogruppe, einer Cyanogruppe, einer Aminogruppe, einer Nitrosogruppe, einer Hydroxygruppe, einer Methoxymethylgruppe, einer *tert*-Butylgruppe, einer *iso*-Propylgruppe, einer C₁-C₆-Alkylgruppe, einer C₁-C₆-Alkyloxygruppe, einer C₁-C₆-Hydroxyalkylgruppe, einer C₁-C₆-Alkylcarbonsäuregruppe, einer C₁-C₆-Alkylcarbonsäureestergruppe, einer C₁-C₆-Alkylsulfonsäuregruppe, einer C₁-C₆-Alkylsulfonsäureestergruppe, oder einer -N-(L)-B⁺-Gruppe sind;
**L** für eine C₁-C₆ Alkylengruppe steht und
**B⁺** a) eine aromatische, heterozyklische quaternäre Ammonium- verbindung, vorzugsweise eine quaternäre Verbindung des N-Methylimidazols, N-Allylimidazols, 2-Ethylimidazols oder 1,2-Dimethylimidazols oder eine quaternäre Verbindung des Pyridins, 4-Dimethylaminopyridins, Pyrimidins, Pyrazols, N-Methyl-pyrazols oder Chinolins, oder
b) eine nicht-aromatische heterozyklische quaternäre Ammoniumverbindung, insbesondere eine quaternäre Verbindung des N-Methyl-morpholins, N-Ethylmorpholins, 1-Methylpiperidins, oder
c) eine quaternäre Alkylammoniumverbindung oder Arylammoniumverbindung der Formel NRₐR_{b}R_{c}, wobei **Rₐ**, **R_{b}**, und **R_{c}** unabhängig voneinander einen Benzylrest, einen Phenylrest oder einen C₁-bis C₆-Alkylrest, insbesondere einen Methylrest, einen Ethylrest, einen Propylrest, einen Isopropylrest oder einen Butylrest bedeuten, wobei die vorgenannten Alkylreste unsubstituiert oder mit einer oder mehreren Hydroxylgruppen oder Aminogruppen substituiert sein können, oder
d) eine quaternäre Phosphoniumgruppe, beispielsweise eine Tributylphosphoniumgruppe, insbesondere aber eine Trimethylammoniumgruppe oder eine Triethylammoniumgruppe darstellt; **R9** gleich einer verzweigten oder linearen C₁-C₆-Alkylgruppe, einer verzweigten oder linearen C₂-C₄-Hydroxyalkylgruppe oder einer verzweigten oder linearen C₄-C₆-Polyhydroxyalkylgruppe ist; und **X⁻** ein Anion, beispielsweise ein Sulfatanion, ein Phosphatanion, ein Hydrogenphosphatanion, ein Oxalatanion, ein Formiatanion, ein Acetatanion, ein Zitratanion, ein Tartratanion, ein Malonatanion, ein Pyruvatanion oder ein Iodidanion ist, wobei das Chloridion, Bromidion und Methylsulfatanion besonders bevorzugt sind; wobei mindestens einer der Reste **R1**, **R2** und **R3** mindestens eine kationische Gruppe aufweist.

Als geeignete kationische Direktzieher der allgemeinen Formel (I) können beispielsweise genannt werden:

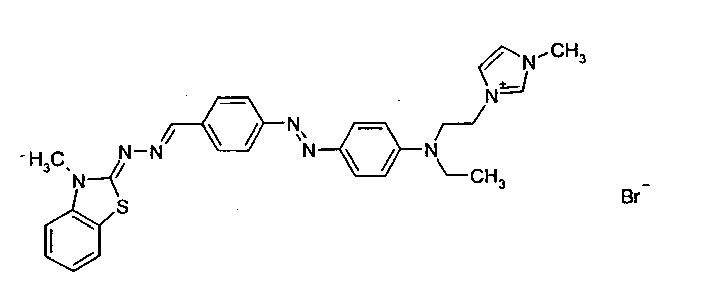

3-(2-{Ethyl-4-[(*E*)-(4-{(*E*)-[(2*Z*)-2-(3-methyl-1,3-benzothiazol-2(3*H*)-yliden)hydrazono]methyl}phenyl)diazenyl]anilino}ethyl)-1-methyl-1*H-*imidazol-3-ium-bromid;

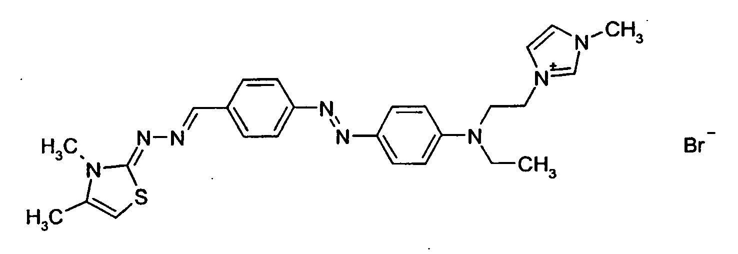

3-{2-[4-[(*E*)-(4-{(*E*)-[(2*Z*)-2-(3,4-Dimethyl-1,3-thiazol-2(3*H*)-yliden)-hydrazono]methyl}phenyl)diazenyl](ethyl)anilino]ethyl}-1-methyl-1*H-*imidazol-3-ium-bromid;

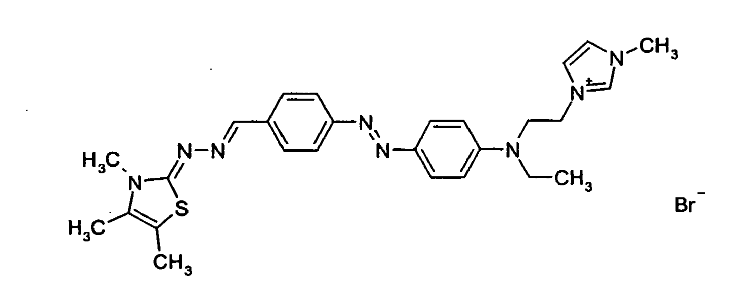

3-(2-{Ethyl-4-[(*E*)-(4-{(*E*)-[(2*Z*)-2-(3,4,5-trimethyl-1,3-thiazol-2(3*H*)-yliden)hydrazono]methyl}phenyl)diazenyl]anilino}ethyl)-1-methyl-1*H-*imidazol-3-ium-bromid;

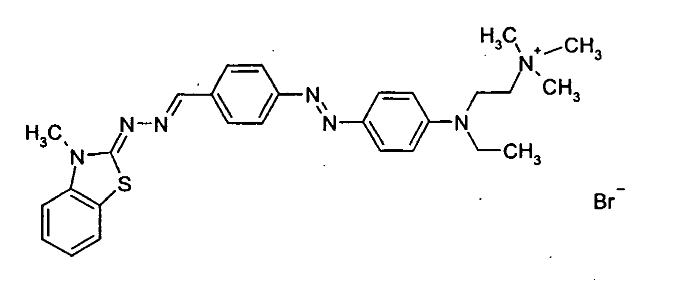

2-{Ethyl-4-[(*E*)-(4-{(*E*)-[(2*Z*)-2-(3-methyl-1,3-benzothiazol-2(3*H*)-yliden)hydrazono]methyl}phenyl)diazenyl]anilino}-*N,N,N-*trimethylethanaminiumbromid;

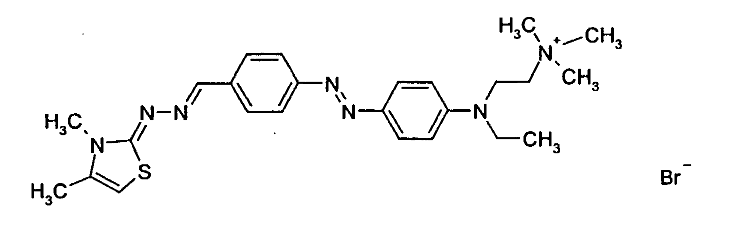

2-[4-[(*E*)-(4-{(*E*)-[(2*Z*)-2-(3,4-Dimethyl-1,3-thiazol-2(3*H*)-yliden)-hydrazono]methyl}phenyl)diazenyl](ethyl)anilino]-*N,N,N-*trimethylethanaminiumbromid;

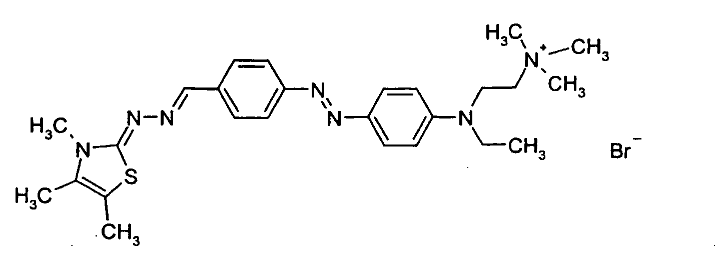

2-{Ethyl-4-[(*E*)-(4-{(*E*)-[(2*Z*)-2-(3,4,5-trimethyl-1,3-thiazol-2(3*H*)-yliden)hydrazono]methyl}phenyl)diazenyl]anilino}-*N,N,N-*trimethylethanaminiumbromid;

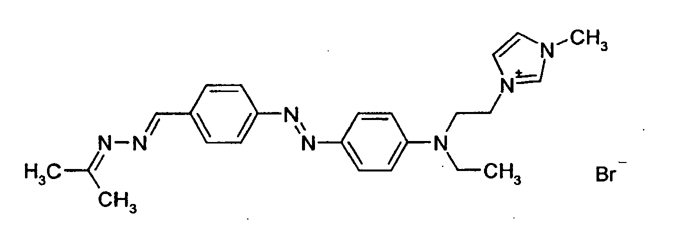

3-(2-{Ethyl-4-[(*E*)-(4-{(*E*)-[(1-methylethyliden)hydrazono]methyl}phenyl)-diazenyl]anilino}ethyl)-1-methyl-1*H*-imidazol-3-ium-bromid;

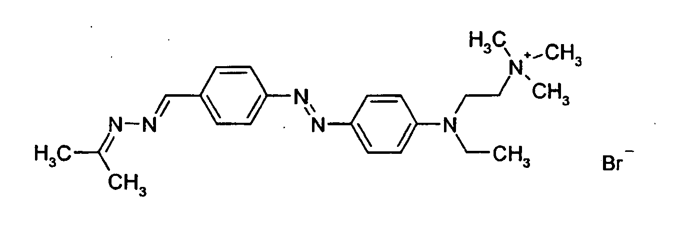

2-{Ethyl-4-[(*E*)-(4-{(*E*)-[(1-methylethyliden)hydrazono]methyl}phenyl)-diazenyl]anilino}-*N,N,N*-trimethylethanaminiumbromid;

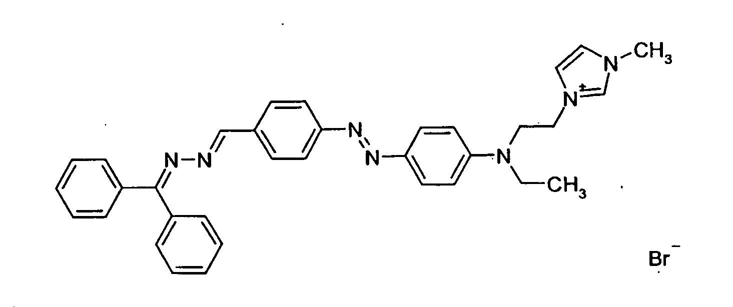

3-{2-[4-[(*E*)-(4-{(*E*)-[(Diphenylmethylen)hydrazono]methyl}phenyl)-diazenyl](ethyl)anilino]ethyl}-1-methyl-1*H*-imidazol-3-ium-bromid;

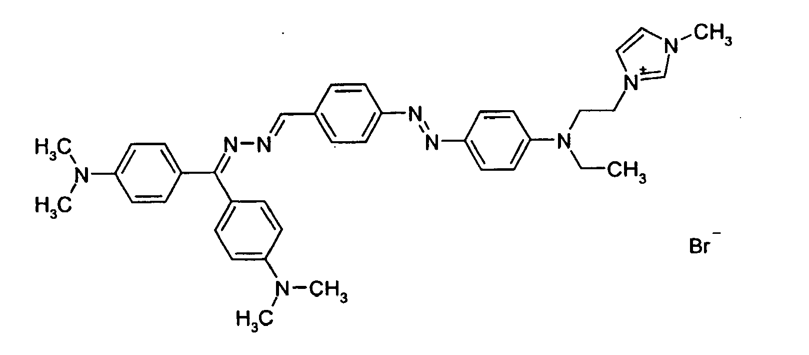

3-{2-[4-((*E*)-{4-[(*E*)-({Bis[4-(dimethylamino)phenyl]methylen}hydrazono)-methyl]phenyl}diazenyl)(ethyl)anilino]ethyl}-1-methyl-1*H*-imidazol-3-iumbromid;

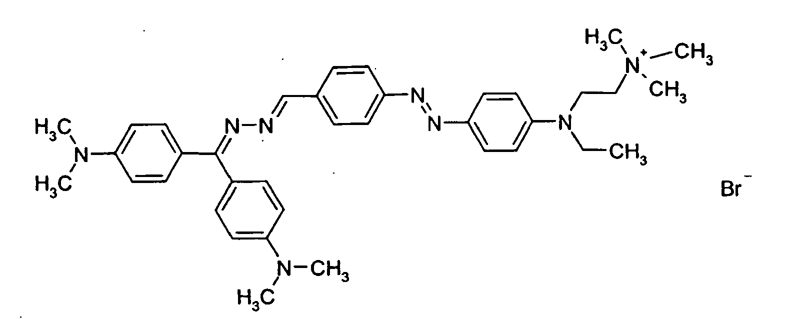

2-[4-((*E*)-{4-[(*E*)-({Bis[4-(dimethylamino)phenyl]methylene}hydrazono)-methyl]phenyl}diazenyl)(ethyl)anilino]-*N,N,N*-trimethylethanaminiumbromid;

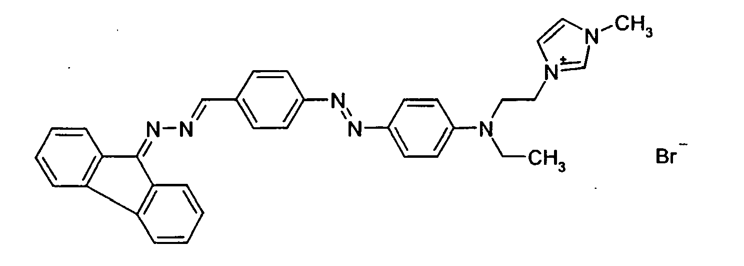

3-{2-[Ethyl-4-((*E*)-{4-[(*E*)-(9*H*-fluoren-9-ylidenhydrazono)methyl]phenyl}-diazenyl)anilino]ethyl}-1-methyl-1H-imidazol-3-ium-bromid;

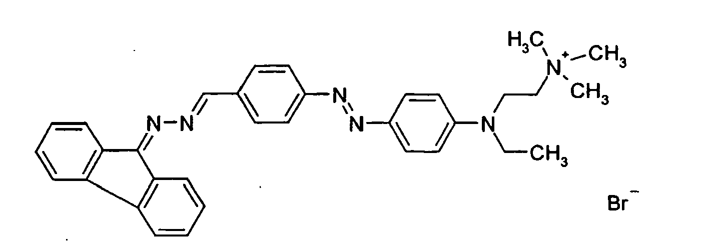

2-[Ethyl-4-((*E*)-{4-[(*E*)-(9*H*-fluoren-9-ylidenhydrazono)methyl]phenyl}-diazenyl)anilino]-*N,N,N*-trimethylethanaminiumbromid;

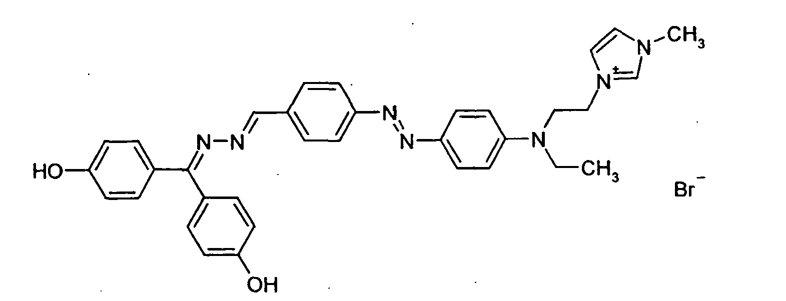

3-{2-[4-{(*E*)-[4-((*E*)-{[Bis(4-hydroxyphenyl)methylen]hydrazono}methyl)-phenyl]diazenyl}(ethyl)anilino]ethyl}-1-methyl-1*H*-imidazol-3-ium-bromid;

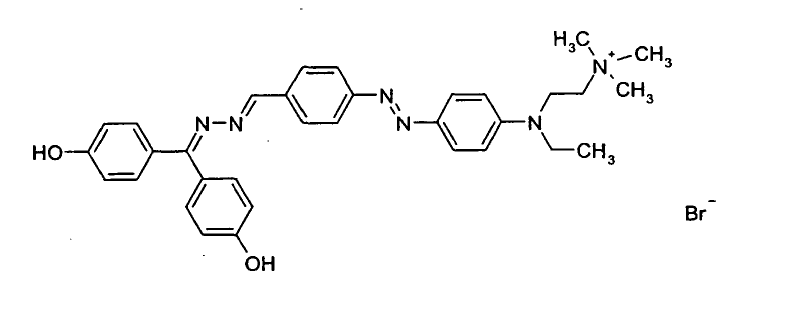

2-[4-{(*E*)4-((*E*)-{[Bis(4-hydroxyphenyl)methylen]hydrazono}methyl)-phenyl]diazenyl}(ethyl)anilino]-*N,N,N*-trimethylethanaminiumbromid;

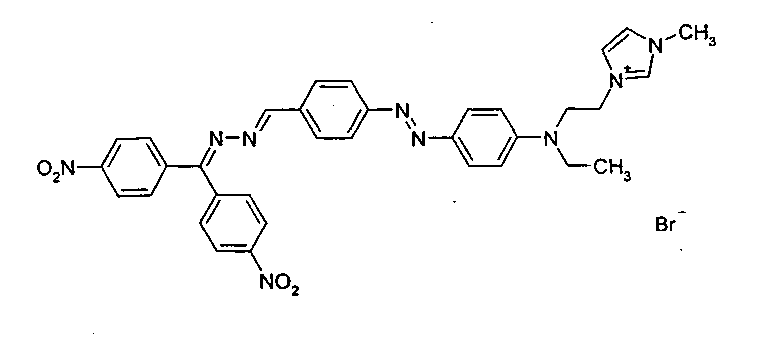

3-{2-[4-{(*E*)-[4-((*E*)-{[Bis(4-nitrophenyl)methylen]hydrazono}methyl)-phenyl]diazenyl}(ethyl)anilino]ethyl}-1-methyl-1*H*-imidazol-3-ium-bromid;

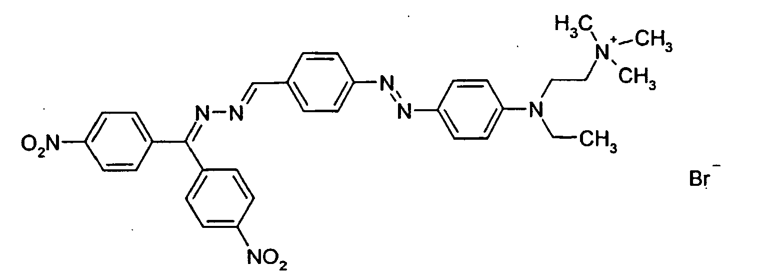

2-[4-{(*E*)-[4-((*E*)-{[Bis(4-nitrophenyl)methylen]hydrazono}methyl)-phenyl]diazenyl}(ethyl)anilino]-*N,N,N*-trimethylethanaminiumbromid;

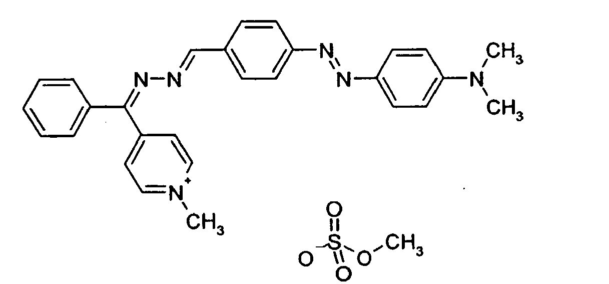

4-[(*Z*)-[(2*E*)-2-(4-{(*E*)-[4-(Dimethylamino)phenyl]diazenyl}benzyliden)-hydrazono](phenyl)methyl]-1-methylpyridinium-methylsulfat;

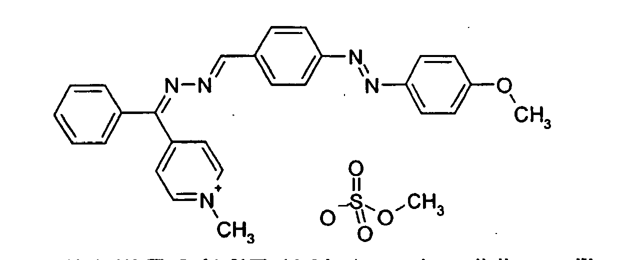

4-[(*Z*)-((2*E*)-2-{4-[(*E*)-(4-Methoxyphenyl)diazenyl]benzyliden}-hydrazono)(phenyl)methyl]-1-methylpyridinium-methylsulfat; und

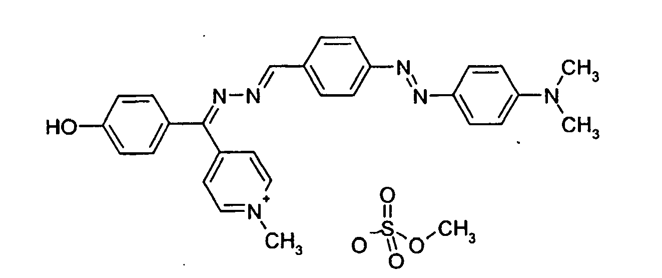

4-[(*Z*)-[(2*E*)-2-(4-{(*E*)-[4-(Dimethylamino)phenyl]diazenyl}-benzyliden)hydrazono](4-hydroxyphenyl)methyl]-1-methylpyridinium-methylsulfat.

Bevorzugte Verbindungen der allgemeinen Formel (I) sind: 3-(2-{Ethyl-4-[(*E*)-(4-{(*E*)-[(2*Z*)-2-(3-methyl-1,3-benzothiazol-2(3*H*)-yliden)hydrazono]methyl}phenyl)-diazenyl]anilino}ethyl)-1-methyl-1*H-*imidazol-3-ium-bromid; 3-{2-[4-[(*E*)-(4-{(*E*)-[(2*Z*)-2-(3,4-Dimethyl-1,3-thiazol-2(3*H*)-yliden)hydrazono]methyl}phenyl)-diazenyl](ethyl)anilino]-ethyl}-1-methyl-1*H*-imidazol-3-ium-bromid; 3-(2-{Ethyl-4-[(*E*)-(4-{(*E*)-[(2*E*)-2-(3,4,5-trimethyl-1,3-thiazol-2(3*H*)-yliden)hydrazono]methyl}-phenyl)diazenyl]anilino}ethyl)-1-methyl-1*H*-imidazol-3-ium-bromid; 2-{Ethyl-4-[(*E*)-(4-{(*E*)-[(2*Z*)-2-(3-methyl-1,3-benzothiazol-2(3H)-yliden)-hydrazono]methyl}-phenyl)diazenyl]anilino}-*N,N,N*-trimethylethanaminiumbromid; 2-[4-[(*E*)-(4-{(*E*)-[(2*Z*)-2-(3,4-Dimethyl-1,3-thiazol-2(3*H*)-yliden)-hydrazono]methyl}phenyl)diazenyl]-(ethyl)anilino]*-N,N,N*-trimethylethanaminiumbromid; 2-{Ethyl-4-[(*E*)-(4-{(*E*)-[(2*Z*)-2-(3,4,5-trimethyl-1,3-thiazol-2(3*H*)-yliden)hydrazono]methyl}phenyl)diazenyl]anilino}-*N,N,N*-trimethylethanaminiumbromid; 3-{2-[4-((*E*)-{4-[(*E*)-({Bis[4-(dimethylamino)phenyl]-methylen}hydrazono)methyl]phenyl}diazenyl)(ethyl)anilino]-ethyl}-1-methyl-1*H*-imidazol-3-ium-bromid und 3-{2-[Ethyl-4-((*E*)-{4-[(*E*)-(9*H-*fluoren-9-ylidenhydrazono)-methyl]phenyl}diazenyl)anilino]ethyl}-1-methyl-1*H*-imidazol-3-ium-bromid.

Die erfindungsgemäßen Farbstoffderivate der allgemeinen Formel (I) sind durch Standardoperationen aus kommerziell erhältlichen oder leicht herstellbaren Komponenten zugänglich.

Nach einem allgemeinen Verfahren nach Y. Mori (Chem. Pharm. Bull. 1981, 29 (5), 1439-1442) lassen sich Azofarbstoffe mit paraständigen Formylsubstituenten herstellen. Die Kondensation dieser Azofarbstoffe in essigsaurem Medium mit diversen Hydrazonkomponenten liefert in guten Ausbeuten die entsprechenden Azoazinfarbstoffe. Ein beispielhafter Syntheseweg ist in Schema 1 dargestellt.

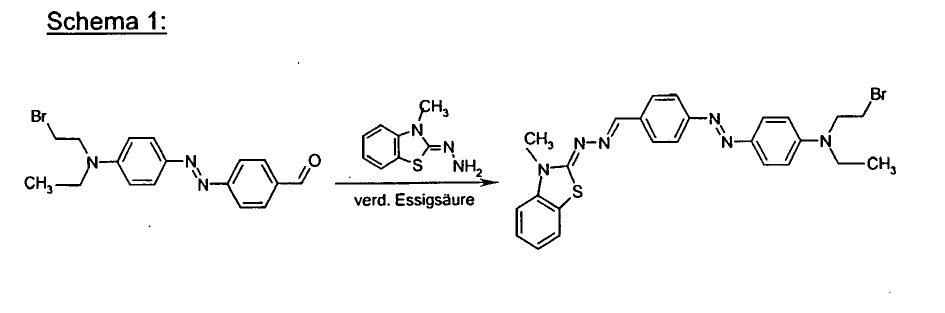

Der kationische Farbstoff wird im abschließenden Schritt durch Substitution einer geeigneten Abgangsgruppe mit *N*-Nucleophilen (z.B. *N*-Methylimidazol) in dipolar aprotischen Lösungsmitteln (z.B. Acetonitril) erhalten (Schema 2).

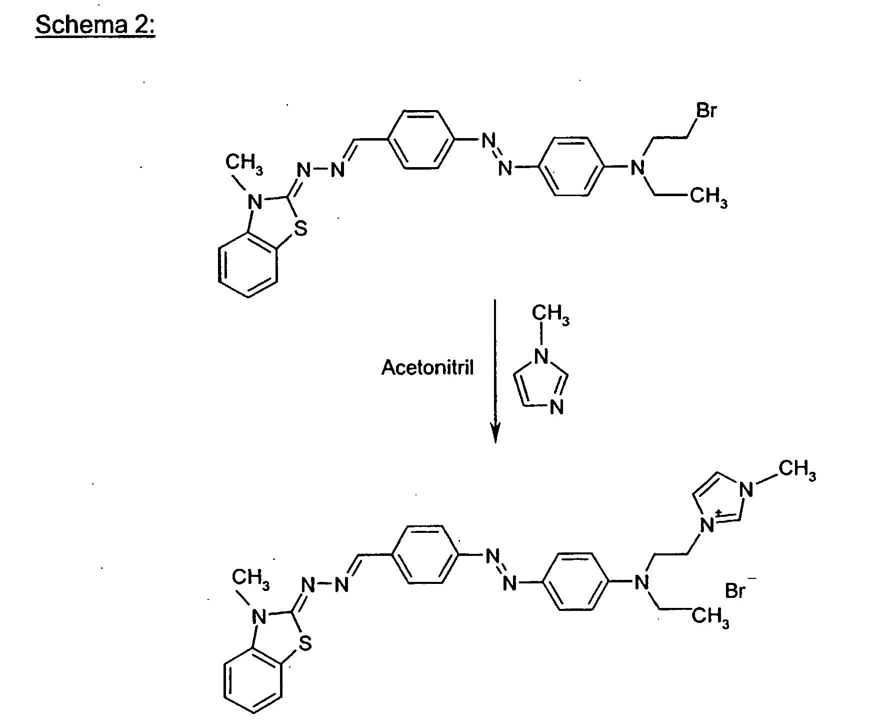

Die erfindungsgemäßen kationischen Azoazinfarbstoffe der allgemeinen Formel (I) ermöglichen eine gleichmäßige Färbung von Fasermaterialien, insbesondere menschlichen Haaren, mit einer guten Stabilität gegen Licht, Schweiss und einer besonderen Stabilität gegen Shampoonieren. Die erfindungsgemäßen Verbindungen der allgemeinen Formel (I) zeigen eine intensive, brillante Färbung von Keratinfasern, insbesondere von menschlichen Haaren, aber auch Wolle, Pelzen oder anderen Fasermaterialien, unter schonenden Bedingungen.

Ein weiterer Gegenstand der vorliegenden Erfindung sind daher
**(a)** ein Mittel zum nicht-oxidativen Färben von Keratinfasern, wie zum Beispiel Haaren, insbesondere menschlichen Haaren,
**(b)** ein Mittel zum gleichzeitigen Aufhellen und Färben von Keratinfasern, das neben dem Farbstoff der Formel (I) ein Oxidationsmittel enthält, und
**(c)** ein oxidatives Färbemittel für Keratinfasern auf der Basis mindestens einer Oxidationsfarbstoffvorstufe,
   wobei die Mittel **(a), (b)** und **(c)** dadurch gekennzeichnet sind, dass sie jeweils mindestens einen kationischen Azoazinfarbstoff der allgemeinen Formel (I) enthalten,

Die kationischen Azoazinfarbstoffe der allgemeinen Formel (I) sind in dem erfindungsgemäßen Färbemittel vorzugsweise in einer Menge von 0,01 bis 10 Gewichtsprozent, insbesondere 0,1 bis 8 Gewichtsprozent, enthalten.

Das erfindungsgemäße Färbemittel **(a)** kann neben den Farbstoffen der allgemeinen Formel (I) zusätzlich noch weitere bekannte, direktfärbende Farbstoffe aus der Gruppe bestehend aus Nitrofarbstoffen, Azofarbstoffen, Athrachinonfarbstoffen, Triphenylmethanfarbstoffen und basischen oder sauren Farbstoffen, alleine oder im Gemisch miteinander, enthalten, wie zum Beispiel 1,4-Bis[(2-hydroxy-ethyl)amino]-2-nitrobenzol, 1-(2-Hydroxyethyl)amino-2-nitro-4-[di(2-hydroxyethyl)amino]-benzol, (HC Blue No. 2), 1-Amino-3-methyl-4-[(2-hydroxyethyl)amino]-6-nitrobenzol, (HC Violet No. 1), 4-[Ethyl-(2-hydroxy-ethyl)amino]-1-[(2-hydroxyethyl)amino]-2-nitrobenzol-hydrochlorid (HC Blue No. 12), 1-[(2,3-Dihydroxypropyl)amino]-4-[methyl-(2-hydroxyethyl)-amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol, (HC Violet No. 2); 1-Amino-4-[(2-hydroxyethyl)amino]-2-nitrobenzol (HC Red No. 7), 2-Amino-4,6-dinitrophenol, 1,4-Diamino-2-nitrobenzol (CI76070), 4-Amino-2-nitro-diphenylamin (HC Red No. 1), 1-Amino-4-[di(2-hydroxy-ethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxy-ethyl)amino]-2-nitrobenzol (HC Red No. 3), 4-Amino-2-nitro-1-((prop-2-en-1-yl)amino)-benzol, 4-Amino-3-nitrophenol, 4-[(2-Hydroxyethyl)amino]-3-nitrophenol, 4-[(2-Nitrophenyl)amino]phenol (HC Orange No. 1), 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol, (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] - 2-nitrobenzol (HC Red No. 11), 2-[(2-Hydroxyethyl)amino]-4,6-dinitrophenol, 4-Ethylamino-3-nitrobenzoesäure, 2-[(4-Amino-2-nitrophenyl)-amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 3-Amino-6-(methylamino)-2-nitropyridin, 1,2,3,4-Tetrahydro-6-nitrochinoxalin, 7-Amino-3,4-dihydro-6-nitro-2H-1,4-benzoxazin (HC Red No. 14), 1,2-Diamino-4-nitrobenzol (CI76020), 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol, (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)-mino]-3-nitro-1-trifluormethyl-benzol, (HC Yellow No. 6), 1-Chlor-2,4-bis[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 2,4-Dinitro-1-hydroxy-naphthalin, 1,4-Di[(2,3-dihydroxypropyl)amino]-9,10-anthrachinon, 1,4-Di[(2-hydroxyethyl)amino]-9,10-anthrachinon (CI61545, Disperse Blue 23), 1-Amino-4-hydroxy-9,10-anthrachinon (CI60710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (CI75470, Natural Red 4), 1-[(3-Aminopropyl)amino]-9,10-anthrachinon (HC Red No. 8), 1,4-Diamino-9,10-anthrachinon (CI61100, Disperse Violet No. 1), 1-Amino-4-(methylamino)-9,10-anthrachinon (CI61105, Disperse Violet No. 4, Solvent Violet No. 12), N-(6-((3-Chlor-4-(methylamino)phenyl)imino)-4-methyl-3-oxo-1,4-cyclohexadien-1-yl)harnstoff (HC Red No. 9), 2-((4-(Di(2-hydroxyethyl)amino)phenyl)-amino)-5-((2-hydroxyethyl)amino)-2,5-cyclohexadien-1,4-dion (HC Green No. 1), 2-Hydroxy-1,4-naphthochinon (CI75480, Natural Orange No. 6), 1,2-Dihydro-2-(1,3-dihydro-3-oxo-2H-indol-2-yliden)-3H-indol-3-on (CI73000), 1,3-Bis(dicyanomethylen)indan, Di[4-(diethylamino)phenyl][4-(ethylamino)naphthyl]carbenium-chlorid (CI42595; Basic Blue No. 7), Di[4-(dimethylamino)phenyl][4-(phenylamino)naphthyl]carbeniumchlorid (CI44045; Basic Blue No. 26), Basic Blue No. 77, 8-Amino-2-brom-5-hydroxy-4-imino-6-[(3-(trimethylammonio)phenyl)amino]-1(4H)-naphthalinon-chlorid (CI56059; Basic Blue No. 99), Tri(4-amino-3-methylphenyl)carbenium-chlorid (CI42520; Basic Violet No. 2), Di(4-aminophenyl)(4-amino-3-methylphenyl)carbenium-chlorid (CI42510; Basic Violet No. 14), 1-[(4-Aminophenyl)azo]-7-(trimethylammonio)-2-naphtholchlorid (CI12250; Basic Brown No. 16), 3-[(4-Amino-2,5-dimethoxyphenyl)azo]-N,N,N-trimethylbenzolaminium-chlorid (CI112605, Basic Orange No. 69), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethylammonio)-2-naphthol-chlorid (CI12251; Basic Brown No. 17), 2-((4-Aminophenyl)azo)-1,3-dimethyl-1H-imidazol-3-ium-chlorid (Basic Orange No. 31), 3,7-Diamino-2,8-dimethyl-5-phenylphenazinium-chlorid (CI50240; Basic Red No. 2), 1,4-Dimethyl-5-[(4-(dimethylamino)phenyl)-azo]-1,2,4-triazolium-chlorid (CI11055; Basic Red No. 22), 1,3-Dimethyl-2-((4-dimethylamino)phenyl)azo-1 H-imidazol-3-ium-chlorid (Basic Red No. 51), 2-Hydroxy-1-[(2-methoxyphenyl)azo]-7-(trimethylammonio)-naphthalin-chlorid (CI12245; Basic Red No. 76), 3-Methyl-1-phenyl-4-[(3-(trimethylammonio)phenyl)azo]-pyrazol-5-on-chlorid (CI12719; Basic Yellow No. 57), 1-Methyl-4-((methyl-phenylhydrazono)methyl)-pyridinium-methylsulfat (Basic Yellow No. 87), 1-(2-Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid, 1-[Di(2-hydroxyethyl)amino]-3-methyl-4-[(4-nitrophenyl)azo]-benzol (CI11210, Disperse Red No. 17), 1-[Di(2-hydroxyethyl)amino]-4-[(4-nitrophenyl)azo]-benzol, (Disperse Black No. 9), 4-[(4-Aminophenyl)azo]-1-[di(2-hydroxyethyl)-amino]-3-methylbenzol (HC Yellow No. 7) oder 2,6-Diamino-3-[(pyridin-3-yl)azo]-pyridin und 2-((4-(Ethyl(2-hydroxyethyl)amino)-2-methylphenyl)azo)-5-nitro-1,3-thiazol (CI111935; Disperse Blue No. 106).

Das erfindungsgemäße Färbemittel **(b),** das sich durch einen Gehalt an einem Oxidationsmittel, vorzugsweise Wasserstoffperoxid auszeichnet, kann neben den Farbstoffen der allgemeinen Formel (I) zusätzlich noch weitere oxidationsstabile direktfärbende Farbstoffe, wie zum Beispiel 3-(2',6'-Diaminopyridyl-3'-azo)-pyridin (= 2,6-Diamino-3-((pyridin-3-yl)azo)pyridin, 2-((4-(Ethyl(2-hydroxyethyl)-amino)-2-methylphenyl)azo-5-nitro-1,3-thiazol (Disperse Blue 106), N,N-Di(2-hydroxyethyl)-3-methyl-4-((4-nitrophenyl)azo)-anilin (Disperse Red 17, CI 11210), 3-Diethylamino-7-(4-dimethylaminophenylazo)-5-phenyl-phenaziniumchlorid (CI 11050), 4-(2-Thiazolylazo)-resorcin, 4-(((4-Phenylamino)azo)benzosulfonsäurenatriumsalz (Orange IV), 1-((3-Aminopropyl)amino)-9,10-anthracendion (HC Red No. 8), 3',3",4,5,5',5",6,7-Octabromphenolsulfonphtalein (Tetrabromphenol Blue), 1-((4-Amino-3,5-dimethylphenyl)-(2,6-dichlorphenyl)-methylen)-3,5-dimethyl-4-imino-2,5-cyclohexadien-Phosphorsäure (1:1) (Basic Blue 77), 3',3",5',5"-Tetrabrom-m-kresolsulfonphthalein, 2,4-Dinitro-1-naphthol-7-sulfonsäure-Dinatriumsalz (Acid Yellow 1, CL 10316), 4-[2'-Hydroxy-1'-naphthyl)azo]-benzosulfonsäure-Natriumsalz (Acid Orange 7, CL 15510), 3',6'-Dihydroxy-2',4',5',7'-tetraiodospiro-[isobenzo-furan-1(3H), 9'(9H)-xanthen]-3-on-Dinatriumsalz (Acid Red 51, CL 45430), 6-Hydroxy-5-((2-methoxy-5-methyl-4-sulfophenyl)azo)-2-Naphthalin-sulfonsäuredinatriumsalz (FD&C Red 40, CL 16035), 2,4-Dinitro-1-naphthol-Natriumsalz (Acid Yellow 24; CL 10315), 2',4',5',7'-tetrabrom-4,5,6,7-tetrachlor-3',6'-dihydroxy-Spiro(isobenzofuran-1(3H), 9'[9H]xanthen]-3-on-dinatriumsalz (Acid Red 92; CL 45410), 4-(2-Hydroxy-1-naphthylazo)-3-methyl-benzolsulfonsäurenatriumsalz (Acid Orange 8, CL 15575), 2-Amino-1,4-naphthalindion, Dithizon (1,5-Diphenylthiocarbazon), N-(2-Hydroxyethyl))-2-nitro-4-trifluormethyl)anilin (HC Yellow 13), N-(2-hydroxyethyl)-4-nitro-anilin und 4-Chlor-N-(2,3-dihydroxypropyl)-2-nitro-anilin, enthalten.

Die vorgenannten direktziehenden Farbstoffe können in einer Gesamtmenge von etwa 0,01 bis 4 Gewichtsprozent enthalten sein, wobei der Gesamtgehalt an Farbstoffen in dem erfindungsgemäßen Färbemittel vorzugsweise etwa 0,01 bis 10 Gewichtsprozent, insbesondere 0,1 bis 5 Gewichtsprozent, beträgt.

Das erfindungsgemäße Oxidationsfärbemittel **(c),** das vor der Anwendung mit einem Oxidationsmittel (insbesondere Wasserstoffperoxid oder dessen Additionsverbindungen) vermischt wird, enthält neben den Farbstoffen der allgemeinen Formel (I) zusätzlich Oxidationsfarbstoffvorstufen. Als geeignete Oxidationsfarbstoffvorstufen können beispielsweise die folgenden Entwicklersubstanzen und Kupplersubstanzen und mit sich selbst kuppelnden Verbindungen genannt werden:
(i) Entwicklersubstanzen: 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)-amino]-anilin, 4-[Di(2-hydroxyethyl)amino]-anilin, 4-[Di(2-hydroxyethyl)amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxy-ethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)-methyl]-1H-pyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol, 2-Amino-5-methyl-phenol, allein oder im Gemisch miteinander.
(ii) Kupplersubstanzen: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxybenzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxy-ethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxy-ethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion, allein oder im Gemisch miteinander.
(iii) Mit sich selbst kuppelnde Verbindungen: 2-Amino-5-methylphenol, 2-Amino-6-methylphenol, 2-Amino-5-ethoxyphenol oder 2-Propylamino-5-aminopyridin.

Die Gesamtmenge der in dem erfindungsgemäßen Färbemittel **(c)** enthaltenen Oxidationsfarbstoffvorstufen beträgt etwa 0,01 bis 12 Gewichtsprozent, insbesondere etwa 0,2 bis 6 Gewichtsprozent.

Das erfindungsgemäße Färbemittel **(a), (b)** oder **(c)** kann weiterhin alle für derartige Zubereitungen bekannten und üblichen Zusatzstoffe, beispielsweise Parfümöle, Komplexbildner, Wachse, Konservierungsstoffe, Verdicker, Alginate, Guar Gum, haarpflegende Substanzen, wie zum Beispiel kationische Polymere oder Lanolinderivate, oder anionische, nichtionische, amphotere oder kationische oberflächenaktive Substanzen enthalten. Vorzugsweise werden amphotere oder nichtionische oberflächenaktive Substanzen, beispielsweise Betaintenside, Propoinate und Glycinate, wie zum Beispiel Cocoamphoglycinate oder Cocoamphdiglycinate, ethoxylierte Tenside mit 1 bis 1000 Ethylenoxid-Einheiten, vorzugsweise mit 1 bis 300 Ethylenoxid-Einheiten, wie zum Beispiel Glyceridalkoxylate, beispielsweise mit 25 Ethylenoxid-Einheiten ethoxyliertes Rizinusöl, Polyglycolamide, ethoxylierte Alkohole und ethoxylierte Fettalkohole (Fettalkoholalkoxylate) und ethoxylierte Fettsäurezuckerester, insbesondere ethoxylierte Sorbitanfettsäureester, eingesetzt. Die vorgenannten Bestandteile werden in den für solche Zwecke üblichen Mengen verwendet, zum Beispiel die oberflächenaktiven Substanzen in einer Konzentration von 0,1 bis 30 Gewichtsprozent, und die Pflegestoffe in einer Menge von 0,1 bis 5 Gewichtsprozent.
Das erfindungsgemässe Färbemittel kann, insbesondere wenn es ein Haarfärbemittel ist, in Form einer wässrigen oder wässrig-alkoholischen Lösung, einer Creme, eines Gels, einer Emulsion oder eines Aerosolschaumes vorliegen, wobei das Haarfärbemittel sowohl in Form eines Einkomponentenpräparates als auch in Form eines Mehrkomponentenpräparates, beispielsweise in Form eines Zweikomponentenpräparates, bei dem das jeweilige Farbstoffderivat der allgemeinen Formel (I) getrennt von den übrigen Bestandteilen abgepackt wird und die Herstellung des gebrauchsfertigen Haarfärbemittels erst unmittelbar vor der Anwendung durch Vermischen der beiden Komponenten erfolgt, konfektioniert sein kann.

Die erfindungsgemäßen Färbemittel **(a), (b)** und **(c)** weisen einen pH von etwa 2 bis 10, vorzugsweise etwa 5 bis10, und insbesondere einem neutralen bis basischen pH-Wert von etwa 7 bis 10, auf. Zur Einstellung des erfindungsgemäßen pH-Wertes sind sowohl organische als auch anorganische Säuren oder Basen geeignet.

Als geeignete Säuren sind insbesondere die folgenden Säuren zu nennen: α-Hydroxycarbonsäuren, wie zum Beispiel Glycolsäure, Milchsäure, Weinsäure, Zitronensäure oder Äpfelsäure, Ascorbinsäure, Gluconsäurelacton, Essigsäure, Salzsäure oder Phosphorsäure, sowie Mischungen dieser Säure. Als geeignete Basen sind inbesonders Natriumcarbonat, Natriumhydrogencarbonat, Alkanolamine, beispielsweise Monoethanolamin oder Triethanolamin, Ammoniak, Aminomethylpropanol und Natriumhydroxid zu nennen.

Die Anwendung des erfindungsgemäßen Färbemittels erfolgt in der Regel indem man eine für die Haarfärbung ausreichende Menge, je nach Haarlänge etwa 30 bis 120 Gramm, des Haarfärbemittels auf das Haar aufträgt, das Haarfaärbemittel bei etwa 15 bis 45 Grad Celsius etwa 1 bis 60 Minuten, vorzugsweise 5 bis 30 Minuten, einwirken lässt, das Haar anschließend gründlich mit Wasser ausspült, gegebenenfalls mit einem Shampoo wäscht und abschließend trocknet.

Das vorstehend beschriebene Färbemittel kann weiterhin -sofern keine Oxidationsmittel der Färbemasse zugesetzt werden- für kosmetische Mittel übliche natürliche oder synthetische Polymere beziehungsweise modifizierte Polymere natürlichen Urprungs enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, basische Polymerisate von Estern der Polyacrylsäure, Polymethylacrylsäure und Aminoalkohole, beispielsweise deren Salze oder Quaternisierungsprodukte, Polyacrylnitril, Polyvinylacetate sowie Copolymerisate aus derartigen Verbindungen, wie zum Beispiel Polyvinylpyrrolidon-Vinylacetat, erwähnt; während als natürliche Polymere oder modifizierte natürliche Polymere beispielsweise Chitosan (entacetyliertes Chitin) oder Chitosanderivate eingesetzt werden können.

Die vorgenannten Polymere können in dem erfindungsgemäßen Färbemittel **(a)** in der für solche Mittel üblichen Mengen, insbesondere in einer Menge von etwa 1 bis 5 Gewichtsprozent, enthalten sein. Der pH-Wert des erfindungsgemäßen Tönungsfestigers oder Farbfestigers beträgt vorzugsweise etwa 6 bis 9.

Die Anwendung des Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Die erfindungsgemäßen Färbemittel **(a), (b)** und **(c)** ermöglichen eine gleichmäßige, intensive und dauerhafte Färbung von Keratinfasern (beispielsweise menschlichen Haaren, Wolle oder Pelzen) ohne nennenswerte Anfärbung der Haut beziehungsweise Kopfhaut, wobei diese Färbung auch im Falle des Färbemittels **(a)** fünf und mehr Haarwäschen ohne ein merkliches Verblassen der Haarfarbe überdauert.

Die nachfolgenden Beispiele sollen den Gegenstand der Erfindung näher erläutern, ohne diesen hierauf zu beschränken.

### Beispiele

### Beispiel 1: Darstellung von 4-((E)-{4-[(2-Bromoethyl)(ethyl)amino]-phenyl}diazenyl)-benzaldehyd

4 g (33 mmol) polymerer *p*-Aminobenzaldehyd werden in einer Mischung aus 80 ml 2N Salzsäure und 40 ml Wasser suspendiert und auf 0 °C abgekühlt. Anschießend tropft man 35 ml einer Lösung von 2,3 g (33 mmol) Natriumnitrit in 8 ml Wasser innerhalb 30 Minuten unter weiterem Abkühlen bis -3 °C zu. Zu der so erhaltenen braunen Diazoniumsalzlösung gibt man nun unter stetiger Kühlung portionsweise (innerhalb 45 Minuten) 7,53 g (33 mmol) *N*-(2-Bromoethyl)-*N*-ethylanilin. Unter weiterer Kühlung rührt man noch eine Stunde und giesst den Ansatz sodann auf Eis. Das Reaktionsgemisch wird anschließend langsam mit 30%iger Natronlauge (ca. 40 ml) neutralisiert, 30 Minuten lang bei Raumtemperatur gerührt und der sich abscheidende Niederschlag abfiltriert, mit Wasser gewaschen und im Vakuum getrocknet. Das Rohprodukt wird abschließend über Kieselgel mit der 1:2-Mischung von Heptan und Essigester chromatographiert.
Ausbeute: 5,75 g (49% der Theorie) orangerotes Pulver
¹H-NMR (d₆-DMSO/300 MHz): δ = 1,16 (t, 3H, methyl), 3,57 (q, 2H, methylen), 3,66-3,68 (m, 2H, methylen), 3,81-3,84 (m, 2H, methylen), 6,90 (d, 2H, *J*= 9,3 Hz, phenyl), 7,84 (d, 2H, *J*= 9,3 Hz, phenyl), 7,93 (d, 2H, *J*= 8,4 Hz, phenyl), 8,05 (d, 2H, *J*= 8,7 Hz, phenyl), 10,07 (s, 1H, formyl).

### Beispiel 2: Darstellung von 3-(2-{Ethyl-4-[(E)-(4-{(E)-[(2Z)-2-(3-methyl-1,3-benzothiazol-2(3H)-yliden)hydrazono]methyl}phenyl)-diazenyl]anilino}-ethyl)-1-methyl-1H-imidazol-3-ium-bromid

### Stufe 1: Darstellung von 4-((E)-{[4-[(2-Bromoethyl)(ethyl)amino]-phenyl}diazenyl)-benzaldehyd((2Z)-3-methyl-1,3-benzothiazol-2(3H)-yliden)-hydrazon

0,2 g (0,55 mmol) 4-((E)-{4-[(2-Bromoethyl)(ethyl)amino]phenyl}diazenyl)-benzaldehyd und 0,13 g (0,55 mmol) *N*-Methyl-benzothiazoliumhydrazonhydrochlorid (MBTH) werden bei Raumtemperatur in 10% Essigsäure 60 Minuten lang gerührt. Anschließend wird der Ansatz mit 50 ml Wasser verdünnt und mit Eis gekühlt.
Die Reaktionsmischung wird mit 2 N Natronlauge neutral eingestellt, der ausgefallene Niederschlag wird sodann abgesaugt, mit Wasser gewaschen und das erhaltene Produkt im Vakuum getrocknet.
Ausbeute: 0,26 g (90% der Theorie) rotes Pulver ¹H-NMR (d₆-DMSO/300 MHz): δ = 1,16 (t, 3H, methyl), 3,52 (q, 2H, methylen), 3,54 (s, 3H, methyl), 3,60-3,68 (m, 2H, methylen), 3,80-3,82 (m, 2H, methylen), 6,88 (d, 2H, *J*= 9,3 Hz, phenyl), 7,11-7,13 (m, 1H, benzothiazol), 7,33-7,36 (m, 2H, benzothiazol), 7,63-7,66 (m, 1H, benzothiazol), 7,82 (d, 2H, *J*= 9,0 Hz, phenyl), 7,86-7,91 (m, 4H, phenyl), 8,46 (s, 1H, olefin).

### Stufe 2: Darstellung von 3-(2-{Ethyl-4-[(E)-(4-{(E)-[(2Z)-2-(3-methyl-1,3-benzothiazol-2(3H)-yliden)hydrazono]methyl}phenyl)diazenyl]anilino}-ethyl)-1-methyl-1H-imidazol-3-ium-bromid

0,2 g (0,38 mmol) 4-((*E*)-{4-[(2-Bromoethyl)(ethyl)amino]phenyl}diazenyl)-benzaldehyd((2*Z*)-3-methyl-1,3-benzothiazol-2(3*H*)-yliden)hydrazon und 0,61 g (7,6 mmol) *N*-Methylimidazol werden 48 Stunden lang in 25 ml Acetonitril unter Rückfluss gekocht. Nach dem Abziehen des Lösungsmittels wird mit Essigester verdünnt und das Produkt mit Wasser extrahiert. Die so isolierte wässrige Phase wird eingedampft, der Rückstand nochmals mit Essigester gewaschen und im Vakuum getrocknet.
- Ausbeute:: 0,16 g (68% der Theorie) orangerotes Pulver
- Schmelpunkt:: 151 °C

### Beispiel 3: Darstellung von 3-{2-[4-[(E)-(4-{(E)-[(2Z)-2-(3,4-Dimethyl-1,3-thiazol-2(3H)-yliden)hydrazono]methyl}phenyl)diazenyl]-(ethyl)anilino]ethyl}-1-methyl-1H-imidazol-3-ium-bromid

### Stufe 1: Darstellung von 4-((E)-{4-[(2-Bromoethyl)(ethyl)amino]-phenyl}diazenyl)-benzaldehyd((2Z)-3,4-dimethyl-1,3-thiazol-2(3H)-yliden)hydrazon

0,43 g (1,18 mmol) 4-((E)-{4-[(2-Bromoethyl)(ethyl)amino]phenyl}-diazenyl)-benzaldehyd und 0,21 g (1,18 mmol) (2Z)-3,4-Dimethyl-1,3-thiazol-2(3H)-onhydrazonhydrochlorid werden bei Raumtemperatur in 10% Essigsäure 60 Minuten lang gerührt. Anschließend wird der Ansatz mit 50 ml Wasser verdünnt und mit Eis gekühlt.
Die Reaktionsmischung wird mit 2 N Natronlauge neutral eingestellt, der ausgefallene Niederschlag wird sodann abgesaugt, mit Wasser gewaschen und das erhaltene Produkt im Vakuum getrocknet.
Ausbeute: 0,46 g (80% der Theorie) granatrotes Pulver ¹H-NMR (CDCl₃/300 MHz): δ = 1,13 (t, 3H, methyl), 2,18 (s, 3H, methyl), 3,41 (q, 2H, methylen), 3,45 (s, 3H, methyl), 3,55-3,61 (m, 2H, methylen), 3,74-3,79 (m, 2H, methylen), 5,77 (s, 1H, thiazol), 6,78 (d, 2H, *J*= 9,0 Hz, phenyl), 7,89-7,85 (m, 4H, phenyl), 7,91 (d, 2H, *J* = 9,0 Hz, phenyl), 8,36 (s, 1H, olefin).

### Stufe 2: Darstellung von 3-{2-[4-[(E)-(4-{(E)-[(2Z)-2-(3,4-Dimethyl-1,3-thiazol-2(3H)-yliden)hydrazono]methyl}phenyl)diazenyl](ethyl)-anilino]ethyl}-1-methyl-1H-imidazol-3-ium-bromid

0,25 g (0,51 mmol) 4-((*E*)-{4-[(2-Bromoethyl)(ethyl)amino]phenyl}-diazenyl)-benzaldehyd((2*Z*)3,4-dimethyl-1,3-thiazol-2(3H)-yliden)-hydrazon und 0,82 g (10,2 mmol) *N*-Methylimidazol werden 48 Stunden lang in 30 ml Acetonitril unter Rückfluss gekocht. Nach dem Abziehen des Lösungsmittels wird der Rückstand mit Essigester verdünnt und das Produkt mit Wasser extrahiert. Die so isolierte wässrige Phase wird eingedampft, der Rückstand nochmals mit Essigester gewaschen und im Vakuum getrocknet.
- Ausbeute:: 0,18 g (59% der Theorie) dunkelrotes Pulver
- Schmelzpunkt:: 132 °C

### Beispiele 4 und 5: Haarfärbemittel

- 2,5 mmol: Farbstoff der allgemeinen Formel (I) gemäß Tabelle 1
- 5,0 g: Ethanol
- 4,0 g: Decylpolyglucose
- 0,2 g: Ethylendiaminotetraessigsäure-Dinatriumsalz-Hydrat
- ad 100,0 g: Wasser, vollentsalzt

Die Färbelösung wird auf einen pH-Wert von 6 bis 7 eingestellt. Die Haarfärbung erfolgt, indem man eine für die Haarfärbung ausreichende Menge des Färbemittels auf das Haar aufträgt, nach einer Einwirkzeit von 30 Minuten bei 40 °C das Haar mit lauwarmen Wasser spült und sodann trocknet.

Die Färbeergebnisse sind in der nachfolgenden **Tabelle 1** zusammengefasst.

**Tabelle 1:**

| **Beispiel Nr.** | **Farbstoff der Formel I (gemäß Beispiel...)** | **Färbeergebnis** |
|---|---|---|
| **4** | 3-(2-{Ethyl-4-[(*E*)-(4-{(*E*)-[(2*Z*)-2-(3-methyl-1,3-benzothiazol-2(3*H*)-yliden)hydrazono]-methyl}phenyl)diazenyl]anilino}-ethyl)-1-methyl-1*H*-imidazol-3-ium-bromid **(2)** | leuchtendes Orange |
| **5** | 3-{2-[4-[(*E*)-(4-{(*E*)-[(2*Z*)2-(3,4-Dimethyl-1,3-thiazol-2(3*H*)-yliden)hydrazono]methyl}-phenyl)diazenyl](ethyl)anilino]ethyl}-1-methyl-1*H*-imidazol-3-ium-bromid **(3)** | Weinrot |

### Beispiele 6 und 7: Haarfärbemittel zum gleichzeitigen Aufhellen und Färben (mit Oxidationsmittel)

- 2,5 mmol: Farbstoff der allgemeinen Formel (I) gemäß Tabelle 2
- 5,0 g: Ethanol
- 4,0 g: Decylpolyglucose
- 0,2 g: Ethylendiaminotetraessigsäure-Dinatriumsalz-Hydrat
- ad 100,0 g: Wasser, vollentsalzt
- 100,0 g: Wasserstoffperoxid (6%ige Lösung in Wasser)

**Tabelle 2:**

| **Beispiel Nr.** | **Farbstoff der Formel I (gemäß Beispiel...)** | **Färbeergebnis** |
|---|---|---|
| **6** | 3-(2-{Ethyl-4-[(*E*)-(4-{(*E*)-[(2*Z*)-2-(3-methyl-1,3-benzothiazol-2(3*H*)-yliden)hydrazono]-methyl}phenyl)diazenyl]anilino}-ethyl)-1-methyl-1*H*-imidazol-3-ium-bromid **(2)** | leuchtendes Orange |
| **7** | 3-{2-[4-[(*E*)-(4-{(*E*)-[(2*Z*)-2-(3,4-Dimethyl-1,3-thiazol-2(3*H*)-yliden)hydrazono]methyl}-phenyl)diazenyl](ethyl)anilino]ethyl}-1-methyl-1*H*-imidazol-3-ium-bromid **(3)** | brilliantes Orangerot |

Alle Prozentangaben stellen -soweit nicht anders angegeben-Gewichtsprozent dar.

## Patentansprüche

1. Kationische Azoazinfarbstoffe der allgemeinen Formel (I), 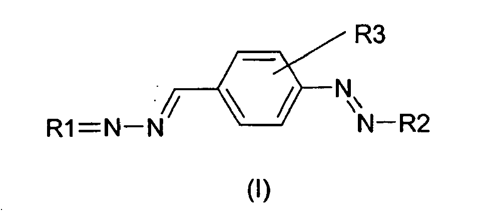 worin
**R1** ausgewählt ist aus Resten der Formeln (II), (III), (IV), (V) und (VI), 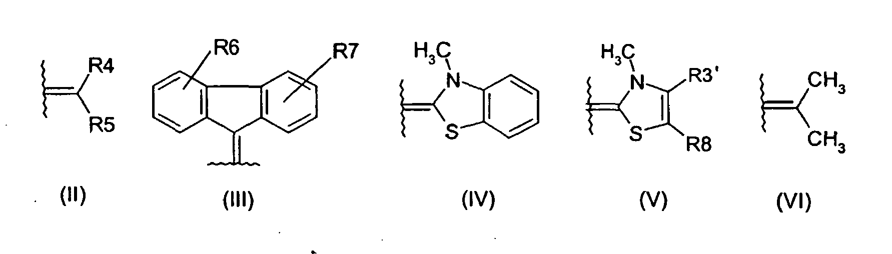 **R2, R4** und **R5** gleich oder verschieden sein können und unabhängig voneinander ausgewählt sind aus Resten der Formeln (VII), (VIII) und (IX); 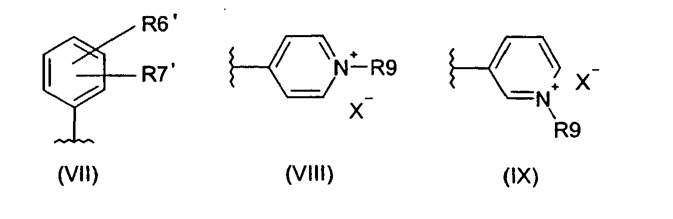 **R3, R3', R6, R6', R7, R7'** und **R8** gleich oder verschieden sein können und unabhängig voneinander gleich Wasserstoff, einer C₁-C₆-Alkylaminogruppe, einer C₁-C₆-*N,N*-Dialkylaminogruppe, einer C₁-C₆-*N,N*-Dihydroxyalkylaminogruppe, einer C₁-C₆-*N*-hydroxyalkyl-*N*-alkylaminogruppe, einer C₁-C₆-Alkyicyanogruppe, einer Nitrogruppe, einer Cyanogruppe, einer Aminogruppe, einer Nitrosogruppe, einer Hydroxygruppe, einer Methoxymethylgruppe, einer *tert*-Butylgruppe, einer *iso*-Propylgruppe, einer C₁-C₆-Alkylgruppe, einer C₁-C₆-Alkyloxygruppe, einer C₁-C₆-Hydroxyalkylgruppe, einer C₁-C₆-Alkylcarbonsäuregruppe, einer C₁-C₆-Alkylcarbonsäureestergruppe, einer C₁-C₆-Alkylsulfonsäuregruppe, einer C₁-C₆-Alkylsulfonsäureestergruppe, oder einer -N-(L)-B⁺ -Gruppe
sind;
**L** für eine C₁-C₆ Alkylengruppe steht und
**B⁺** a) eine aromatische, heterozyklische quaternäre Ammoniumverbindung oder
b) eine nicht-aromatische heterozyklische quaternäre Ammoniumverbindung, oder
c) eine quaternäre Alkylammoniumverbindung oder Arylammoniumverbindung der Formel NRₐR_{b}R_{c}, wobei **Rₐ**, **R_{b}**, und **R_{c}** unabhängig voneinander einen Benzylrest, einen Phenylrest oder einen C₁-bis C₆-Alkylrest, einen Ethylrest, einen Propylrest, einen Isopropylrest oder einen Butylrest bedeuten, wobei die vorgenannten Alkylreste unsubstituiert oder mit einer oder mehreren Hydroxylgruppen oder Aminogruppen substituiert sein können, oder
d) eine quaternäre Phosphoniumgruppe darstellt; **R9** gleich einer verzweigten oder linearen C₁-C₆-Alkylgruppe, einer verzweigten oder linearen C₂-C₄-Hydroxyalkylgruppe oder einer verzweigten oder linearen C₄-C₆-Polyhydroxyalkylgruppe ist; und **X⁻** ein Anion ist;
unter der Bedingung, dass mindestens einer der Reste **R1, R2** und **R3** mindestens eine kationische Gruppe aufweist.

2. Kationischer Azoazinfarbstoff der Formel (I) gemäß Anspruch 1, **dadurch gekennzeichnet, dass** er ausgewählt ist aus 3-(2-{Ethyl-4-[(*E*)-(4-{(*E*)-[(2*Z*)-2-(3-methyl-1,3-benzothiazol-2(3*H*)-yliden)hydrazono]-methyl}phenyl)diazenyl]anilino}ethyl)-1-methyl-1*H*-imidazol-3-ium-bromid; 3-{2-[4-[(*E*)-(4-{(*E*)-[(2*Z*)-2-(3,4-Dimethyl-1,3-thiazol-2(3*H*)-yliden)-hydrazono]methyl}phenyl)diazenyl](ethyl)anilino]ethyl}-1-methyl-1*H-*imidazol-3-ium-bromid; 3-(2-{Ethyl-4-[(*E*)-(4-{(*E*)-[(2*Z*)-2-(3,4,5-trimethyl-1,3-thiazol-2(3*H*)yliden)hydrazono]methyl}phenyl)diazenyl]-anilino}ethyl)-1-methyl-1*H*-imidazol-3-ium-bromid; 2-{Ethyl-4-[(*E*)-(4-{(*E*)-[(2*Z*)-2-(3-methyl-1,3-benzothiazol-2(3*H*)-yliden)hydrazono]methyl}-phenyl)diazenyl]anilino}-*N,N,N*-trimethylethanaminiumbromid; 2-[4-[(*E*)-(4-{(*E*)-[(2*Z*)-2-(3,4-Dimethyl-1,3-thiazol-2(3*H*)-yliden)-hydrazono]methyl}phenyl)diazenyl](ethyl)anilino]-*N,N,N-*trimethylethanaminiumbromid; 2-{Ethyl-4-[(*E*)-(4-{(*E*)-[(2*Z*)-2-(3,4,5-trimethyl-1,3-thiazol-2(3*H*)-yliden)hydrazono]methyl}phenyl)diazenyl]-anilino}-*N,N,N*-trimethylethanaminiumbromid; 3-(2-{Ethyl-4-[(*E*)-(4-{(*E*)-[(1-methylethyliden)hydrazono]methyl}phenyl)-diazenyl]anilino}ethyl)-1-methyl-1*H*-imidazol-3-ium-bromid; 2-{Ethyl-4-[(*E*)-(4-{(*E*)-[(1-methylethyliden)hydrazono]methyl}phenyl)-diazenyl]anilino}-*N,N,N-*trimethylethanaminiumbromid; 3-{2-[4-[(*E*)-(4-{(*E*)-[(Diphenylmethylen)-hydrazono]methyl}phenyl)-diazenyl](ethyl)anilino]ethyl}-1-methyl-1*H-*imidazol-3-ium-methylsulfat; 3-{2-[4-((*E*)-{4-[(*E*)-({Bis[4-(dimethylamino)-phenyl]methylen}hydrazono)-methyl]phenyl}diazenyl)(ethyl)anilino]ethyl}-1-methyl-1*H*-imidazol-3-ium-bromid; 2-[4-((*E*)-{4-[(*E*)-({Bis[4-(dimethylamino)phenyl]methylene}hydrazono)-methyl]phenyl}diazenyl)-(ethyl)anilino]-*N,N,N*-trimethylethanaminiumbromid; 3-{2-[Ethyl-4-((*E*)-{4-[(*E*)-(9*H*-fluoren-9-ylidenhydrazono)methyl]phenyl}-diazenyl)anilino]ethyl}-1-methyl-1*H*-imidazol-3-ium; 2-[Ethyl-4-((*E*)-{4-[(*E*)-(9*H*-fluoren-9-ylidenhydrazono)methyl]phenyl}-diazenyl)anilino]-*N,N,N-*trimethylethanaminiumbromid; 3-{2-[4-{(*E*)-[4-((*E*)-{[Bis(4-hydroxyphenyl)-methylen]hydrazono}methyl)-phenyl]diazenyl}(ethyl)anilino]ethyl}-1-methyl-1*H*-imidazol-3-ium-bromid; 2-[4-{(*E*)-[4-((*E*)-{[Bis(4-hydroxyphenyl)methylen]hydrazono}methyl)-phenyl]diazenyl}-(ethyl)anilino]-*N,N,N*-trimethylethanaminiumbromid; 3-{2-[4-{(*E*)-[4-((*E*)-{[Bis(4-nitrophenyl)methylen]hydrazono}methyl)-phehyl]diazenyl}(ethyl)anilino]ethyl}-1-methyl-1*H*-imidazol-3-ium-bromid; 2-[4-{(*E*)-[4-((*E*)-{[Bis(4-nitrophenyl)methylen]hydrazono}-methyl)-phenyl]diazenyl}(ethyl)anilino]-*N,N,N*-trimethylethanaminium-bromid; 4-[(*Z*)-[(2*E*)-2-(4-{(*E*)-[4-(Dimethylamino)phenyl]diazenyl}-benzyliden)-hydrazono](phenyl)methyl]-1-methylpyridinium-methylsulfat; 4-[(*Z*)-((2*E*)-2-{4-[(*E*)-(4-Methoxyphenyl)diazenyl]benzyliden}-hydrazono)(phenyl)methyl]-1-methylpyridinium-methylsulfat; und 4-[(*Z*)-[(2*E*)-2-(4-{(*E*)-[4-(Dimethylamino)phenyl]diazenyl}-benzyliden)hydrazono](4-hydroxyphenyl)methyl]-1-methylpyridinium-methylsulfat.

3. Mittel zum nicht-oxidativen Färben von Keratinfasern, **dadurch gekennzeichnet, dass** es mindestens einen kationischen Azoazinfarbstoff der allgemeinen Formel (I) gemäß Anspruch 1 oder 2 enthält.

4. Mittel nach Anspruch 3, **dadurch gekennzeichnet, dass** es mindestens ein für kosmetische Mittel übliches natürliches Polymer, synthetisches Polymer oder modifiziertes Polymer natürlichen Ursprungs enthält und in Form eines Tönungsfestigers oder Farbfestigers vorliegt.

5. Mittel zum gleichzeitigen Aufhellen und Färben von Keratinfasern, **dadurch gekennzeichnet, dass** es (i) ein Oxidationsmittel sowie (ii) mindestens einen kationischen Azoazinfarbstoff der allgemeinen Formel (I) gemäß Anspruch 1 oder 2 enthält.

6. Mittel nach Anspruch 5, **dadurch gekennzeichnet, dass** das Oxidationsmittel Wasserstoffperoxid ist.

7. Mittel zum oxidativen Färben von Keratinfasern auf der Basis von Oxidationsfarbstoffvorstufen, **dadurch gekennzeichnet, dass** es mindestens einen kationischen Azoazinfarbstoff der allgemeinen Formel (I) gemäß Anspruch 1 oder 2 enthält.

8. Mittel nach Anspruch 7, **dadurch gekennzeichnet, dass** es 0,01 bis 12 Gewichtsprozent mindestens einer Oxidationsfarbstoffvorstufe enthält.

9. Mittel nach einem der Ansprüche 3 bis 7, **dadurch gekennzeichnet, dass** der kationische Azoazinfarbstoff der allgemeinen Formel (I) in einer Gesamtmenge von 0,01 bis 10 Gewichtsprozent enthalten ist.

10. Mittel nach einem der Ansprüche 3 bis 9, **dadurch gekennzeichnet** es neben dem kationischen Azoazinfarbstoff der allgemeinen Formel (I) zusätzlich mindestens einen weiteren direktfärbenden Farbstoff aus der Gruppe bestehend aus Nitrofarbstoffen, Azofarbstoffen, Anthrachinonfarbstoffen, Triphenylmethanfarbstoffen und basischen oder sauren Farbstoffen enthält.

11. Mittel nach einem der Ansprüche 3 bis 10**, dadurch gekennzeichnet, dass** es einen pH-Wert von 2 bis 11 aufweist.

12. Mittel nach einem der Ansprüche 3 bis 11, **dadurch gekennzeichnet, dass** es ein Haarfärbemittel ist.

## Claims

1. A cationic azo azine dye of the general formula (I), 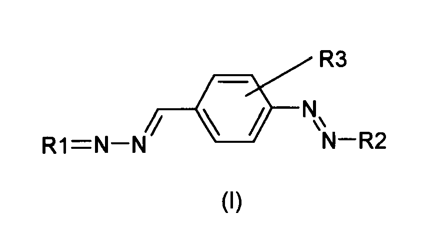 wherein
**R1** is selected from moieties having the formulas (II), (III), (IV), (V) and (VI), 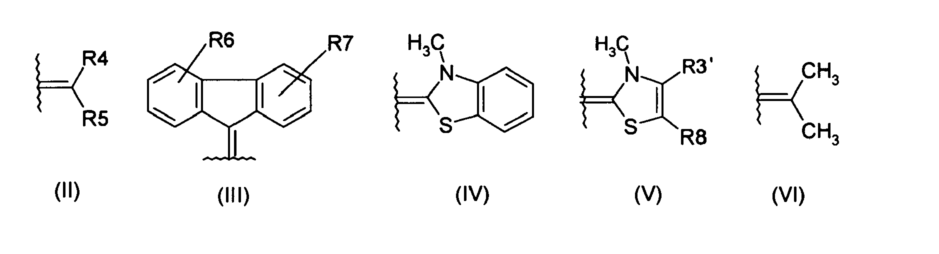 **R2, R4** and **R5** can be the same or different and independently from one another are selected from moieties having the formulas (VII), (VIII) and (IX); 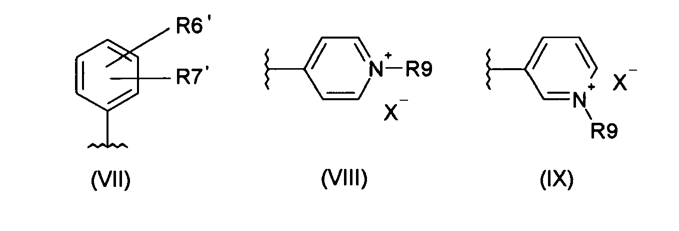 **R3, R3', R6, R6', R7, R7'** and **R8** can be the same or different and independently from one another equal hydrogen, a C₁-C₆-alkylamine group, a C₁-C₆-*N,N*-dialkylamino group, a C₁-C₆-*N,N*-dihydroxyalkylamino group, a C₁-C₆-*N*-hydroxyalkyl-*N-*alkylamino group, a C₁-C₆-alkylcyano group, a nitro group, a cyano group, an amino group, a nitroso group, a hydroxyl group, a methoxymethyl group, a *tert*-butyl group, an *iso*-propyl group, a C₁-C₆-alkyl group, a C₁-C₆-alkyloxy group, a C₁-C₆-hydroxyalkyl group, a C₁-C₆-alkylcarboxylic acid group, a C₁-C₆-alkylcarboxylic acid ester group, a C₁-C₆-alkylsulfonic acid group, a C₁-C₆-alkylsulfonic acid ester group, or an -N-(L)-B⁺ -group; **L** stands for a C₁-C₆ alkylene group and **B⁺** represents a) an aromatic, heterocyclic quaternary ammonium compound or
b) a nonaromatic heterocyclic quaternary ammonium compound, or
c) a quaternary alkylammonium compound or arylammonium compound of the formula NRₐR_{b}R_{c}, where **Rₐ, R_{b},** and **R_{c}** independently from one another indicate a benzyl moiety, a phenyl moiety or a C₁-bis C₆-alkyl moiety, an ethyl moiety, a propyl moiety, an isopropyl moiety or a butyl moiety, where the above-named alkyl moieties can be unsubstituted or substituted with one or more hydroxyl groups or amino groups, or
d) a quaternary phosphonium group; **R9** equals a branched or linear C₁-C₆-alkyl group, a branched or linear C₂-C₄-hydroxyalkyl group or a branched or linear C₄-C₆-polyhydroxyalkyl group; and **X⁻** is an anion; provided that at least one of the moieties **R1, R2** and **R3** will exhibit at least one cationic group.

2. The cationic azo azine dye of formula (I) according to Claim 1, **characterized in that** said dye is selected from among 3-(2-{ethyl-4-[(*E*)-(4-{(*E*)-[(2*Z*)-2-(3-methyl-1,3-benzothiazol-2(3*H*)ylidene)hydrazono]methyl}phenyl)diazenyl]anilino}ethyl)-1-methyl-1*H*-imidazol-3-ium bromide; 3-{2-[4-[(*E*)-(4-{(*E*)-[(2*Z*)-2-(3,4-dimethyl-1,3-thiazol-2(3*H*)ylidene)hydrazono]methyl}phenyl)diazenyl](ethyl)anilino]ethyl}-1-methyl-1*H-*imidazol-3-ium bromide; 3-(2-{ethyl-4-[(*E*)-(4-{(*E*)-[(2*Z*)-2-(3,4,5-trimethyl-1,3-thiazol-2(3*H*)ylidene)hydrazono]methyl}phenyl)diazenyl]anilino}ethyl)-1-methyl-1*H*-imidazol-3-ium bromide; 2-{ethyl-4-[(*E*)-(4-{(*E*)-[(2*Z*)-2-(3-methyl-1,3-benzothiazol-2(3*H*)ylidene)hydrazono]methyl}phenyl)diazenyl]anilino}-*N,N,N-*trimethylethanaminium bromide; 2-[4-[(*E*)-(4-{(*E*)-[(2*Z*)-2-(3,4-dimethyl-1,3-thiazol-2(3*H*)ylidene)hydrazono]methyl}phenyl)diazenyl](ethyl)anilino]-*N,N,N-*trimethylethanaminium bromide; 2-{thyl-4-[(*E*)-(4-[(*E*)-[(2*Z*)-2-(3,4,5-trimethyl-1,3-thiazol-2(3*H*)ylidene)hydrazono]methyl}phenyl)diazenyl]anilino}-*N,N,N-*trimethylethanaminium bromide; 3-(2-{ethyl-4-[(*E*)-(4-{(*E*)-[(1-methylethylidene)hydrazono]methyl}phenyl)diazenyl]anilino}ethyl)-1-methyl-1*H*-imidazol-3-ium bromide; 2-{ethyl-4-[(*E*)-(4-{(*E*)-[(l-methylethylidene)hydrazono]methyl}phenyl)-diazenyl]anilino}-*N,N,N*-trimethylethanaminium bromide; 3-{2-[4-[(*E*)-(4-{(*E*)-[(diphenylmethylidene)hydrazono]methyl}phenyl)diazenyl](ethyl)anilino]ethyl}-1-methyl-1*H*-imidazol-3-ium methyl sulfate; 3-{2-[4-((*E*)-{4-[(*E*)-({bis-[4-(dimethylamino)-phenyl]methylidene}hydrazono)methyl]phenyl}diazenyl)(ethyl)anilino]ethyl}-1-methyl-1*H*-imidazol-3-ium bromide; 2-[4-((*E*)-{4-[(*E*)-({bis-[4-(dimethylamino)phenyl]methylidene}hydrazono)methyl]phenyl}diazenyl)(ethyl)anilino]-*N,N,N*-trimethylethanaminium bromide; 3-{2-[ethyl-4-((*E*)-{4-[(*E*)-(9*H*-fluoren-9-ylidenehydrazono)methyl]phenyl}diazenyl)anilino]ethyl}-1-methyl-1*H*-imidazol-3-ium; 2-[ethyl-4-((*E*)-{4-[(*E*)-(9*H*-fluoren-9-ylidenehydrazono)methyl]phenyl}diazenyl)anilino]-*N,N,N*-trimethylethanaminium bromide; 3-{2-[4-{(*E*)-[4-((*E*)-{[bis-(4-hydroxyphenyl)methylidene]hydrazono}methyl)-phenyl]diazenyl}(ethyl)anilino]ethyl}-1-methyl-1*H*-imidazol-3-ium bromide; 2-[4-{(*E*)-[4-((*E*)-{[bis-(4-hydroxyphenyl)methylidene]hydrazono}methyl)phenyl]diazenyl}(ethyl)-anilino]-*N,N,N*-trimethylethananaminium bromide; 3-{2-[4-{(*E*)-[4-((*E*)-{[bis-(4-nitrophenyl)methylidene]hydrazono}methyl)phenyl]diazenyl}(ethyl)anilino]ethyl}-1-methyl-1*H*-imidazol-3-ium bromide; 2-[4-{(*E*)-[4-((*E*)-{[bis-(4-nitrophenyl)methylidene]hydrazono}methyl)phenyl]diazenyl}(ethyl)anilino]-*N,N,N*-trimethylethanaminium bromide; 4-[(*Z*)-[(2*E*)-2-(4-{(*E*)-[4-(dimethylamino)phenyl]diazenyl}benzylidene)hydrazono]-(phenyl)methyl]-1-methylpyridinium methyl sulfate; 4-[(*Z*)-((2*E*)-2-{4-[(*E*)-(4-methoxyphenyl)diazenyl]benzylidene}hydrazono)(phenyl)methyl]-1-methylpyridinium methyl sulfate; and 4-[(*Z*)-[(2*E*)-2-(4-{(*E*)-[4-(dimethylamino)phenyl]diazenyl}benzylidene)-hydrazono](4-hydroxyphenyl)methyl]-1-methylpyridinium methyl sulfate.

3. An agent for the nonoxidative coloring of keratin fibers, **characterized in that** said agent contains at least one cationic azo azine dye of the general formula (I) according to Claim 1 or 2.

4. The agent according to Claim 3, **characterized in that** said agent contains at least one natural polymer customarily used as a cosmetic agent or a modified polymer of natural origin and is present in the form of a tone sealer or color strengthener.

5. An agent for the simultaneous oxidative brightening and coloring of keratin fibers, **characterized in that** it contains (i) an oxidizing agent, as well as at least (ii) a cationic azo azine dye of the general formula (I) according to Claim 1 or 2.

6. The agent according to Claim 5, **characterized in that** the oxidizing agent is hydrogen peroxide.

7. An agent for the oxidative coloring of keratin fibers based on oxidative dye precursors, **characterized in that** said agent contains at least one cationic azo azine dye of the general formula (I) according to Claim 1 or 2.

8. The agent according to Claim 7, **characterized in that** said agent contains 0.01 to 12% by weight of at least one oxidative dye precursors.

9. The agent according to one of Claims 3 to 7, **characterized in that** the cationic azo azine dye of the general formula (I) is contained in a total amount of from 0.01 to 10% by weight.

10. The agent according to one of Claims 3 to 9, **characterized in that** in addition to the cationic azo azine dye of the general formula (I) said agent contains at least one further direct-penetrating dye from the group that includes nitro dyes, azo dyes, anthraquinone dyes, triphenylmethane dyes and basic or acidic dyes.

11. The agent according to one of Claims 3 to 10, **characterized in that** said agent exhibits a pH value of from 2 to 11.

12. The agent according to one of Claims 3 to 11, **characterized in that** said agent is a hair dye.

## Revendications

1. Teinture azo azine cationique de formule générale (I), 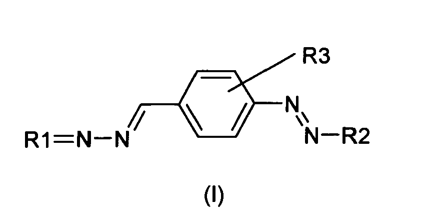 dans laquelle
**R1** est choisi parmi les résidus de formules (II), (III), (IV), (V) et (VI), 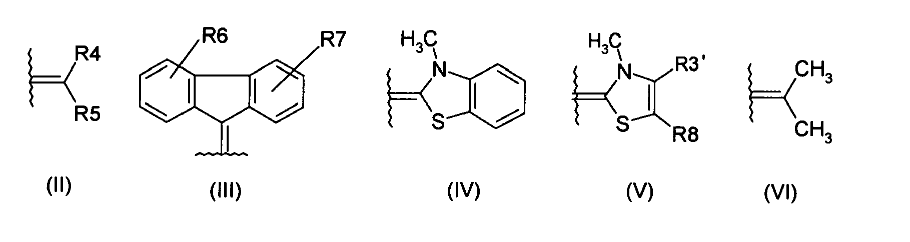 **R2, R4** et **R5** peuvent être identiques ou différents et indépendamment les uns des autres sont choisis parmi les résidus de formules (VII), (VIII) et (IX) ; 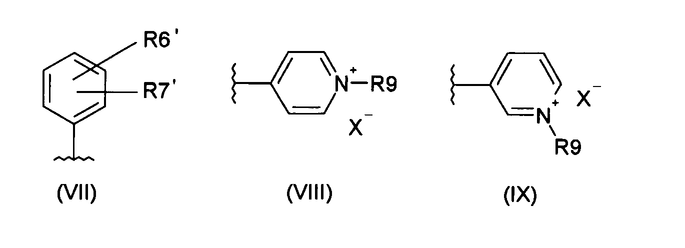 **R3, R3', R6, R6', R7, R7'** et **R8** peuvent être identiques ou différents et indépendamment les uns des autres représentent un hydrogène, un groupe alkylamine en C₁ à C₆, un groupe *N,N*- dialkylamino en C₁ à C₆, un groupe *N,N*-dihydroxyalkylamino en C₁ à C₆, un groupe *N*-hydroxyalkyl-*N*-alkylamino en C₁ à C₆, un groupe alkylcyano en C₁ à C₆, un groupe nitro, un groupe cyano, un groupe amino, un groupe nitroso, un groupe hydroxyle, un groupe méthoxyméthyle, un groupe *tert*-butyle, un groupe *iso*-propyle, un groupe alkyle en C₁ à C₆, un groupe alkyloxy en C₁ à C₆, un groupe hydroxyalkyle en C₁ à C₆, un groupe acide alkylcarboxylique en C₁ à C₆, un groupe ester d'acide alkylcarboxylique en C₁ à C₆, un groupe acide alkylsulfonique en C₁ à C₆, un groupe ester d'acide alkylsulfonique en C₁ à C₆, ou un groupe -N-(L)-B⁺ ;
**L** représente un groupe alkylène en C₁ à C₆ et
**B⁺** représente a) un composé d'ammonium quaternaire hétérocyclique aromatique ou
b) un composé d'ammonium quaternaire hétérocyclique non aromatique, ou
c) un composé d'alkylammonium quaternaire ou un composé d'arylammonium de formule NRₐR_{b}R_{c}, où **Rₐ, R_{b},** et **R_{c}** indépendamment l'un de l'autre indiquent un résidu benzyle, un résidu phényle ou un résidu alkyle en C₁ à C₆, un résidu éthyle, un résidu propyle, un résidu isopropyle ou un résidu butyle, où les résidus alkyle cités précédemment peuvent être non substitués ou substitués avec un ou plusieurs groupes hydroxyle ou groupes amino, ou
d) un groupe phosphonium quaternaire ; **R9** représente un groupe alkyle en C₁ à C₆ ramifié ou linéaire, un groupe hydroxyalkyle en C₂ à C₄ ramifié ou linéaire ou un groupe polyhydroxyalkyle en C₄ à C₆ ramifié ou linéaire ; et
**X⁻** est un anion ;
pour autant qu'au moins un des résidus **R1, R2** et **R3** présentera au moins un groupe cationique.

2. Teinture azo azine cationique de formule (I) selon la revendication 1, **caractérisée en ce que** ladite teinture est choisie parmi le bromure de 3-(2-{éthyl-4-[(*E*)-(4-{(*E*)-[(2*Z*)-2-(3-méthyl-1,3-benzothiazol-2(3*H*)ylidène)hydrazono]méthyl}phényl)diazényl]anilino}éthyl)-1-méthyl-1*H-*imidazol-3-ium ; le bromure de 3-{2-[4-[(*E*)-(4-{(*E*)-[(2*Z*)-2-(3,4-diméthyl-1,3-thiazol-2(3*H*)ylidène)hydrazono]méthyl}phényl)diazényl](éthyl)anilino]éthyl}-1-méthyl-1*H*-imidazol-3-ium ; le bromure de 3-(2-{éthyl-4-[(*E*)-(4-{(*E*)-[(2*Z*)-2-(3,4,5-triméthyl-1,3-thiazol-2(3*H*)ylidène)hydrazono]méthyl}phényl)diazényl]anilino}éthyl)-1-méthyl-1*H-*imidazol-3-ium ; le bromure de 2-{éthyl-4-[(*E*)-(4-{(*E*)-[(2*Z*)-2-(3-méthyl-1,3-benzothiazol-2(3*H*)ylidène)hydrazono]méthyl}phényl)diazényl]anilino}-*N,N,N-*triméthyléthanaminium ; le bromure de 2-[4-[(*E*)-(4-{(*E*)-[(2Z)-2-(3,4-diméthyl-1,3-thiazol-2(3*H*)ylidène)hydrazono]méthyl}phényl)diazényl](éthyl)anilino]-*N,N,N-*triméthyléthanaminium ; le bromure de 2-{éthyl-4-[(*E*)-(4-{(*E*)-[(2*Z*)-2-(3,4,5-triméthyl-1,3-thiazol-2(3*H*)ylidène)hydrazono]méthyl}phényl)diazényl]anilino}-*N,N,N*-triméthyléthanaminium ; le bromure de 3-(2-{éthyl-4-[(*E*)-(4-{(*E*)-[(1-méthyléthylidène)hydrazono]méthyl}phényl)diazényl]anilino}éthyl)-1-méthyl-1*H-*imidazol-3-ium ; le bromure de 2-{éthyl-4-[(*E*)-(4-{(*E*)-[(1-méthyléthylidène)hydrazono]méthyl}phényl)diazényl]anilino}-*N,N,N-*triméthyléthanaminium ; le méthylsulfate de 3-{2-[4-[(*E*)-(4-{(*E*)-[(diphénylméthylidène)hydrazono]méthyl}phényl)diazényl](éthyl)anilino]éthyl}-1-méthyl-1*H*-imidazol-3-ium ; le bromure de 3-{2-[4-((*E*)-{4-[(*E*)-({bis-[4-(diméthylamino)phényl]méthylidène}hydrazono)méthyl]phényl}diazényl)(éthyl)anilin o]éthyl}-1-méthyl-1*H*-imidazol-3-ium ; le bromure de 2-[4-((*E*)-{4-[(*E*)-({bis-[4-(diméthylamino)phényl]méthylidène}hydrazono)méthyl]phényl}diazényl)(éthyl)anilin o]-*N,N,N*-triméthyléthanaminium ; le 3-{2-[éthyl-4-((*E*)-{4-[(*E*)-(9*H*-fluorén-9-ylidènehydrazono)méthyl]phényl}diazényl)anilino]éthyl}-1-méthyl-1*H*-imidazol-3-ium ; le bromure de 2-[éthyl-4-((*E*)-{4-[(*E*)-(9*H*-fluorén-9-ylidènehydrazono)méthyl]phényl}diazényl)anilino]-*N,N,N*-triméthyléthanaminium ; le bromure de 3-{2-[4-{(*E*)-[4-((*E*)-{[bis-(4-hydroxyphényl)méthylidène]hydrazono}méthyl)phényl]diazényl}(éthyl)anilino]éthyl} -1-méthyl-1*H*-imidazol-3-ium ; le bromure de 2-[4-{(*E*)-[4-((*E*)-{[bis-(4-hydroxyphényl)méthylidène]hydrazono}méthyl)phényl]diazényl}(éthyl)anilino]-*N,N,N*-triméthyléthanaminium ; le bromure de 3-{2-[4-{(*E*)-[4-((*E*)-{[bis-(4-nitrophényl)méthylidène]hydrazono}méthyl)phényl]diazényl}(éthyl)anilino]éthyl}-1-méthyl-1*H*-imidazol-3-ium ; le bromure de 2-[4-{(*E*)-[4-((*E*)-{[bis-(4-nitrophényl)méthylidène]hydrazono}méthyl)phényl]diazényl}(éthyl)anilino]-*N,N,N-*triméthyléthanaminium ; le méthylsulfate de 4-[(*Z*)-[(2*E*)-2-(4-{(*E*)-[4-(diméthylamino)phényl]diazényl}benzylidène)hydrazono](phényl)méthyl]-1-méthylpyridinium ; le méthylsulfate de 4-[(*Z*)-((2*E*)-2-{4-[(*E*)-(4-méthoxyphényl)diazényl]benzylidène}hydrazono)(phényl)méthyl]-1-méthylpyridinium ; et le méthylsulfate de 4-[(*Z*)-[(2*E*)-2-(4-{(*E*)-[4-(diméthylamino)phényl]diazényl}benzylidène)hydrazono](4-hydroxyphényl)méthyl]-1-méthylpyridinium.

3. Agent pour la coloration non oxydative de fibres de kératine, **caractérisé en ce que** ledit agent contient au moins une teinture azo azine cationique de formule générale (I) selon la revendication 1 ou 2.

4. Agent selon la revendication 3, **caractérisé en ce que** ledit agent contient au moins un polymère naturel utilisé habituellement en tant qu'agent cosmétique, un polymère synthétrique ou un polymère modifié d'origine naturelle et est présent sous la forme d'un protecteur de teinte ou d'un renforçateur de couleur.

5. Agent pour l'éclaircissement oxydatif et la coloration simultanée de fibres de kératine, **caractérisé en ce qu'**il contient (i) un agent oxydant, ainsi qu'au moins (ii) une teinture azo azine cationique de formule générale (I) selon la revendication 1 ou 2.

6. Agent selon la revendication 5, **caractérisé en ce que** l'agent oxydant est du peroxyde d'hydrogène.

7. Agent pour la coloration oxydative de fibres de kératine basé sur des précurseurs de teinture oxydative, **caractérisé en ce que** ledit agent contient au moins une teinture azo azine cationique de formule générale (I) selon la revendication 1 ou 2.

8. Agent selon la revendication 7, **caractérisé en ce que** ledit agent contient 0,01 à 12 % en poids d'au moins un précurseur de teinture oxydative.

9. Agent selon l'une des revendications 3 à 7, **caractérisé en ce que** la teinture azo azine cationique de formule générale (I) est contenue en une quantité totale allant de 0,01 à 10 % en poids.

10. Agent selon l'une des revendications 3 à 9, **caractérisé en ce qu'**en plus de la teinture azo azine cationique de formule générale (I) ledit agent contient au moins une autre teinture à pénétration directe du groupe qui inclut les teintures nitrées, les teintures azoïques, les teintures anthraquinone, les teintures triphénylméthane et les teintures basiques ou acides.

11. Agent selon l'une des revendications 3 à 10, **caractérisé en ce que** ledit agent présente une valeur de pH allant de 2 à 11.

12. Agent selon l'une des revendications 3 à 11, **caractérisé en ce que** ledit agent est une teinture capillaire.
